# EUROPÄISCHE PATENTSCHRIFT

(11) **EP 2 218 392 B1**
(45) Veröffentlichungstag und Bekanntmachung des Hinweises auf die Patenterteilung: **16.11.2016**
(21) Anmeldenummer: 09002121.3
(22) Anmeldetag: 16.02.2009
(51) Int. Cl.: A61B 5/1455

(54) **STECHSYSTEM**
PIERCING SYSTEM
SYSTÈME DE CONNEXION

(43) Veröffentlichungstag der Anmeldung: 18.08.2010
(73) Patentinhaber: Roche Diabetes Care GmbH, 68305 Mannheim (DE); F. Hoffmann-La Roche AG, 4070 Basel (CH)
(72) Erfinder: Konya, Ahmet, 67165 Waldsee (DE)
(74) Vertreter: Twelmeier Mommer & Partner

(56) Entgegenhaltungen:
- EP-A- 0 254 203
- EP-A- 0 931 507
- EP-A- 1 598 011
- EP-A- 2 030 566
- WO-A-00/40150
- WO-A-2004/041082
- WO-A-2007/041244
- WO-A-2008/131920
- US-A1- 2004 132 167

## Beschreibung

Die Erfindung geht aus von einem Stechsystem mit den im Oberbegriff des Anspruch 1 angegebenen Merkmalen, wie es aus der WO 2008/131920 bekannt ist.

Zu einem derartigen System gehört neben Stech- und Testelementen ein Handgerät mit einem Halter, der bei einem Stich ein Stechelement und ein ihm zugeordnetes Testelement hält, einem Stechantrieb, der bei einem Stich den Halter aus einer Ausgangsposition in eine Einstichposition und zurück in die Ausgangsposition bewegt, einem lichtempfindlichen Sensor zur Untersuchung einer von einem Testelement aufgenommenen Körperflüssigkeitsprobe und einer Lichtquelle zum Beleuchten des Testelements

Derartige Stechsysteme werden beispielsweise von Diabetikern benötigt, die mehrmals täglich ihren Blutzuckerspiegel überprüfen müssen und dafür eine Körperflüssigkeitsprobe, in der Regel Blut oder interstitielle Flüssigkeit benötigen, die aus einer mit einem Stechsystem erzeugten Stichwunde gewonnen wird.

Einen besonders hohen Benutzerkomfort bieten Stechsysteme, bei denen zum Erzeugen einer Stichwunde und dem Aufnehmen einer Probe von einer erzeugten Stichwunde dasselbe Gerät verwendet werden kann. Durch eine automatische Probenaufnahme wird einem Benutzer die Untersuchung einer Körperflüssigkeitsprobe erleichtert, was insbesondere für Personen mit einer durch Alter oder Krankheit eingeschränkten manuellen Beweglichkeit ein wichtiger Vorteil ist. Zudem besteht bei einer automatischen Probenaufnahme eine geringere Gefahr einer Probenkontamination, die zu einer Verfälschung von Messergebnissen führen könnte.

Bei Stechsystemen der eingangs genannten Art, die eine Körperflüssigkeitsprobe mit optischen Mitteln untersuchen, beispielsweise eine photometrische Konzentrationsbestimmung vornehmen, muss Licht mit möglichst geringen Signalverlust und möglichst großem Signalrauschverhältnis von einem Testelement zu einem lichtempfindlichen Sensor geführt werden.

Aufgabe der vorliegenden Erfindung ist es, einen Weg aufzuzeigen, wie dies bei einem Stechsystem der eingangs genannten Art kostengünstig erreicht werden kann.

Diese Aufgabe wird durch ein Stechsystem mit den im Anspruch 1 angegebenen Merkmalen gelöst. Vorteilhafte Weiterbildungen der Erfindung sind Gegenstand von Unteransprüchen.

Bei einem erfindungsgemäßen Stechsystem ist der lichtempfindliche Sensor in einem Gehäuse angeordnet, an dessen Außenseite der Halter in seiner Ausgangsposition anliegt. Durch die erfindungsgemäße Maßnahme kann mit einfachen Mitteln eine präzise Positionierung des Testelements in Bezug auf den Sensor erreicht werden, so dass Messungen mit vorteilhaft geringen Probenvolumina und einer entsprechend kleinen auszuwertenden Fläche des Testelements durchgeführt werden können. Insbesondere kann durch das Gehäuse Störlicht abgeschirmt werden, so dass Messlicht von dem Testelement zu dem Sensor mit geringen Signalverlusten und einem guten Signalrauschverhältnis übertragen werden kann.

Bei einem erfindungsgemäßen Stechsystem können Stechelemente verwendet werden, die integral mit Testelementen ausgebildet sind, wie dies beispielsweise aus der EP 1 360 935 B1 bekannt ist. Derartige Stechelemente haben üblicherweise einen Kapillarkanal, der zu einem auf einem Körper des Stechelements angeordneten Testelement, beispielsweise einem aufgeklebten Testfeld mit Nachweisreagenzien, führt. Möglich ist es jedoch auch, separate Stechelemente und Testelemente zu verwenden, die durch einen geeigneten Transportmechanismus in dem Stechgerät, insbesondere in dem Halter, zusammengeführt werden, so dass durch einen Stich eine Körperflüssigkeitsprobe von einem Stechelement aufgenommen und auf ein Testelement übertragen werden können. Ein derartiges System ist beispielsweise aus der WO 2005/107596 A2 bekannt.

Eine vorteilhafte Weiterbildung der Erfindung sieht vor, dass das Gehäuse an seiner Außenseite eine Führung aufweist, die bei einer Stichbewegung den Halter führt. Auf diese Weise lässt sich erreichen, dass der Halter in seiner Ausgangsposition stets reproduzierbar und mit hoher Genauigkeit eine vorgegebene Position in Bezug auf das Gehäuse und damit in Bezug auf den in dem Gehäuse angeordneten Sensor einnimmt. Dies hat den Vorteil, dass ein von dem Halter gehaltenes Testelement ebenfalls mit hoher Präzision eine definierte Ausgangsposition für eine Messung einnimmt und deshalb der von dem Sensor erfasste Bereich auf eine vorteilhaft kleine Fläche begrenzt werden kann. Dies ist insbesondere für Messungen mit kleinen Probenvolumina ein wichtiger Vorteil, da kleine Probenvolumina nur kleine Flächen benetzen können und das Signalrauschverhältnis im allgemeinen um so besser ist, je besser der von dem Sensor erfasste Bereich mit der für die Messung relevanten Fläche des Testelements übereinstimmt. Bevorzugt ist die Führung eine Linearführung, beispielsweise eine Schwalbenschwanzführung oder eine Schienenführung.

Eine weitere vorteilhafte Weiterbildung der Erfindung sieht vor, dass auch die Lichtquelle zum Beleuchten des Testelements in dem Gehäuse angeordnet ist. Auf diese Weise lässt sich eine störende Beeinflussung der Messung durch Umgebungslicht weitestgehend ausschließen, da im Wesentlichen der gesamte Strahlengang von der Lichtquelle zu dem Testelement und von dem Testelement zu dem Sensor abgeschirmt in dem Gehäuse verlaufen kann. Prinzipiell ist es aber auch möglich, die Lichtquelle außerhalb des Gehäuses anzuordnen und ein Testelement beispielsweise in Transmissionen zu untersuchen.

Bevorzugt ist in dem Gehäuse mindestens ein optisches Element, beispielsweise eine oder mehrere Linsen und/ oder einer oder mehrere Spiegel. Auf diese Weise kann in dem Gehäuse ein optischer Strahlengang für eine präzise Messung vorgegeben werden, der den im Gerät zur Verfügung stehenden Bauraum optimal ausnutzt.

Bevorzugt ist das den Sensor enthaltende Gehäuse ortsfest im Inneren des Geräts angeordnet. Prinzipiell ist es aber auch möglich, den Sensor in einem mehrteiligen Gehäuse anzuordnen, das einen Gehäuseteil aufweist, der sich bei einem Stich zusammen mit dem Halter bewegt.

Weitere Einzelheiten und Vorteile der Erfindung werden an einem Ausführungsbeispiel unter Bezugnahem auf die beigefügten Zeichnungen erläutert. Es zeigen:
- Figur 1:: eine schematische Darstellung eines erfindungsgemäßen Stechsystems mit geöffnetem Gerätegehäuse;
- Figur 2:: eine schematische Darstellung eines Stechelements mit einem Testelement;
- Figur 3:: den Halter des dargestellten Stechsystems mit einem Stechelement und einem Gehäuse, in dem ein Sensor angeordnet ist;
- Figur 4:: eine Explosionsvorstellung zu Figur 3;
- Figur 5:: eine Schnittansicht des den Sensor enthaltenden Gehäuses
- Figur 6:: eine weitere Schnittansicht des den Sensor enthaltenden Gehäuses.

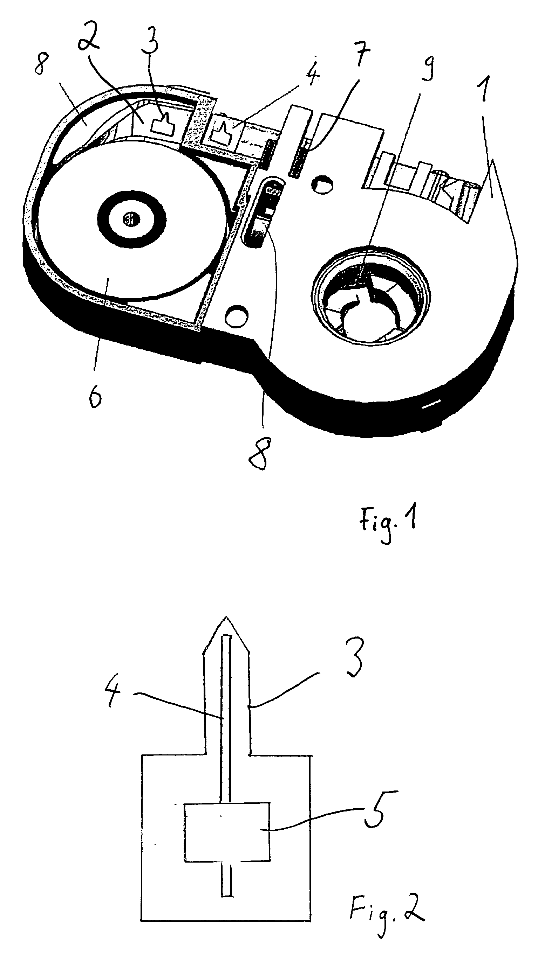

Figur 1 zeigt im Ausführungsbeispiel eines Stechsystems 1 mit geöffnetem Gerätegehäuse. In dem Gerätegehäuse ist ein Trägerband 2 angeordnet, das Stech- und Testelemente trägt. Die in Figur 2 beispielhaft dargestellten Stechelemente 3 haben jeweils einen Kanal 4, um Körperflüssigkeit zu einem Testelement 5 mit Nachweisreagenzien zur fotometrischen Bestimmung einer Analytkonzentration zu transportieren.

Das Trägerband 2 ist mit unbenutzten Stech- und Testelementen zu einer Rolle 6 aufgewickelt und wird von dort zu dem Halter 7, der bei einem Stich ein Stechelement 3 mit dem zugeordneten Testelement 5 hält, geführt. Der Halter 7 ist mit einem Stechantrieb 8 gekoppelt, der bei einem Stich den Halter 7 aus einer Ausgangsposition in eine Einstichposition, die den Umkehrpunkt einer Stich- und Rückführbewegung darstellt, und wieder zurück in die Ausgangsposition bewegt.

Das Trägerband 2 kann mit benutzten Stech- und Testelementen mittels einer Wickeleinrichtung 9 aufgewickelt werden. Durch diesen Aufwickelvorgang wird ein Bandtransport bewirkt, so dass unbenutzte Stech- und Testelemente nacheinander zu dem Halter 7 geführt werden können.

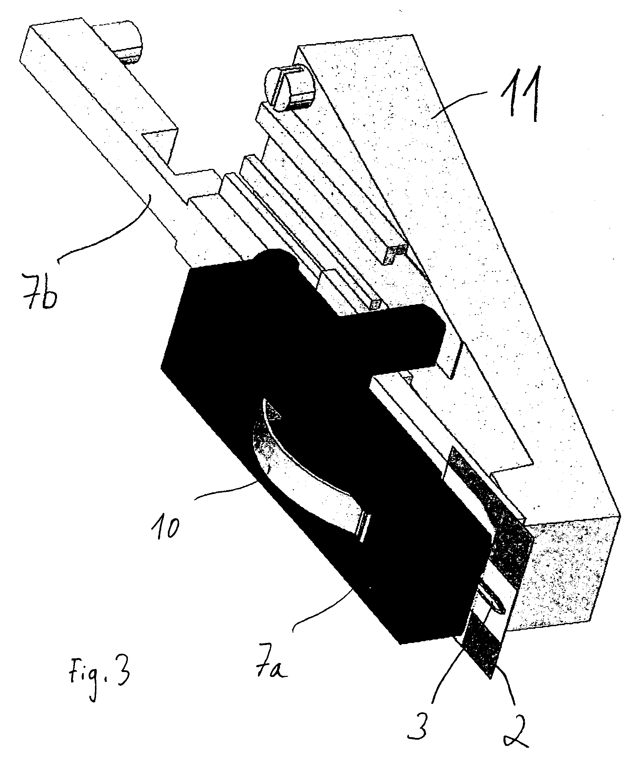

Figur 3 zeigt eine Detailansicht des Halters 7 mit einem Stechelement 3 auf einem Abschnitt des Trägerbands 2. Der Halter besteht aus zwei Halteelementen 7a, 7b, die relativ zu einander schwenkbar sind und bei einem Stich zwischen sich ein Stechelement 3 mit einem ihm zugeordneten Testelement 5 klemmend halten. Um einen Bandtransport zu ermöglichen, können die beiden Halteelemente 7a, 7b gegeneinander um eine Achse verschwenkt werden, so dass sich zwischen ihnen ein Spalt öffnet. Wenn ein neues Stechelement 3 mit einem dazugehörenden Testelement in seine Gebrauchsposition gebracht wurde, wird der Spalt durch eine entsprechende Bewegung der beiden Halteelemente 7a, 7b wieder geschlossen. Die Schließbewegung wird durch die Rückstellkraft einer Feder 10 bewirkt.

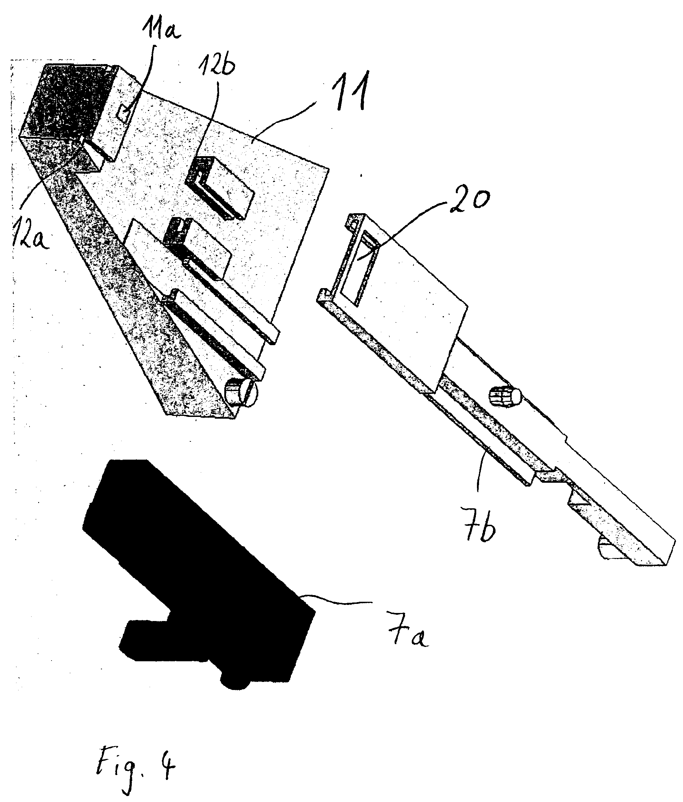

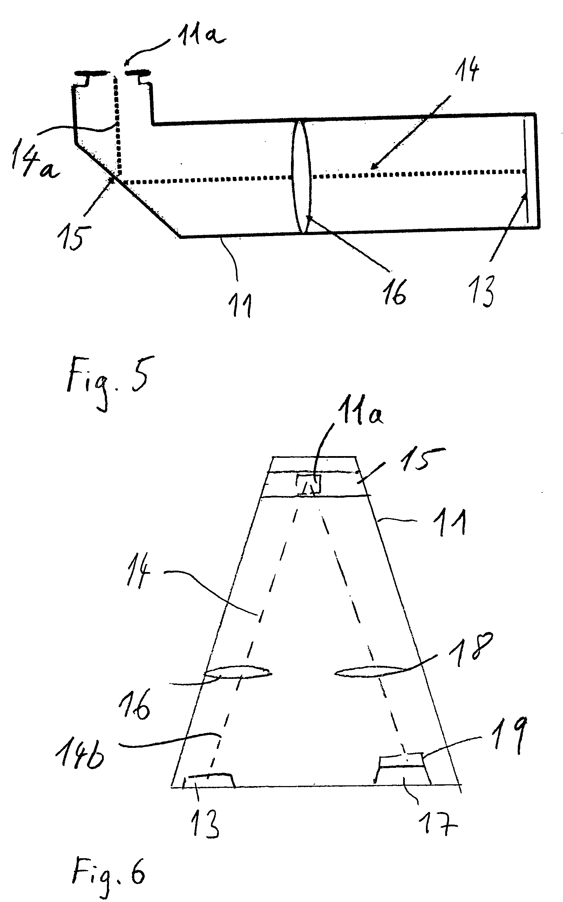

Der Halter 7 liegt in seiner in Figur 3 dargestellten Ausgangsstellung an der Außenseite eines Gehäuses 11 an, in dem ein lichtempfindlicher Sensor 13 zur Untersuchung einer von einem Testelement 5 aufgenommenen Körperflüssigkeitsprobe angeordnet ist. Ein Schnitt durch das Gehäuse 11 ist in Figur 5 gezeigt. Das Gehäuse 11 hat ein Fenster 11a, durch welches Licht von dem Testelement 5 zu dem Sensor 13 gelangt. Dieses Fenster 11a ist bevorzugt als eine Öffnung in dem Gehäuse 11 ausgeführt. Möglich ist es jedoch auch, das Fenster 11a als einen transparenten Gehäuseabschnitt auszubilden. Das an dem Gehäuse 11 anliegende Halteelement 7b hat ebenfalls ein Fenster 20, das in Figur 4 dargestellt ist. Das Fenster 20 ist bevorzugt eine Aussparung und kann vorteilhaft größer als das Fender 11a ausgebildet werden, mit dem es in der Ausgangslage des Halters 7 fluchtet.

Das lichtdichte Gehäuse 11 schirmt Störlicht ab, so dass darin Messungen mit einem guten Signalrauschverhältnis vorgenommen werden können. Das Gehäuse 11 weist an seiner Außenseite Führungen 12a, 12b, bei dem dargestellten Ausführungsbeispiel Nuten bzw. Schwalbenschwanzführungen, auf, die bei einer Stichbewegung den Halter 7 führen. Auf diese Weise wird einerseits eine geradlinige und deshalb schmerzarme Stichbewegung bewirkt, andererseits wird vorteilhaft die Position des Testelements 5 in der Ausgangsposition des Halters 7 in Bezug auf das Gehäuse 11 und damit in Bezug auf den darin angeordneten Sensor 13 sehr präzise vorgegeben.

Der in dem Gehäuse 11 angeordnete Sensor 13 und ein dazugehörender Strahlengang 14 mit optischen Elementen 15, 16, die schematisch in Figur 5 als ein Spiegel 15 und eine Linse 16 dargestellt sind, kann deshalb in der Herstellung sehr präzise auf die Position eines von dem Halter 7 in seiner Ausgangsstellung gehaltenen Testelements 5 mit einer aufgenommenen Körperflüssigkeitsprobe eingerichtet werden, so dass auch mit sehr kleinen Probevolumina von wenigen nl, beispielsweise 20 nl bis 200 nl, eine präzise Messung möglich ist.

In dem sich in Stichrichtung V-förmig verjüngenden Gehäuse ist auch eine in Figur 6 schematisch dargestellte Lichtquelle 17 zum Beleuchten des Testelements 5, beispielsweise eine LED angeordnet. Von der Lichtquelle 17 ausgesandtes Licht wird über optische Elemente, beispielsweise eine Linse 18, eine Blende 19 und den Spiegel 15, zu dem Testelement und von dort auf einem V-förmigen Strahlengang zu dem Sensor 13 gelenkt, wie dies in Figur 6 angedeutet ist.

Der Strahlengang 14 verläuft in dem Gehäuse 11 gewinkelt. Ein von dem zu untersuchenden Testelement 5 ausgehender Anfangsabschnitt 14a des Strahlengangs verläuft quer zur Stichrichtung, bevorzugt senkrecht zu der Stichrichtung, in welcher der Halter 7 bei einem Stich bewegt wird. Ein Endabschnitt 14 b des Strahlengangs 14 verläuft entlang der Stichrichtung und schließt mit dieser bevorzugt einen spitzen Winkel ein.

### Bezugszahlen

- 1: Stechsystem
- 2: Trägerband
- 3: Stechelement
- 4: Kanal
- 5: Testelement
- 6: Rolle
- 7: Halter
- 7a: Halteelement
- 7b: Halteelement
- 8: Stechantrieb
- 9: Wickeleinrichtung
- 10: Feder
- 11: Gehäuse
- 11a: Fenster
- 12 12a: Führungen
- 12b: Führungen
- 13: Sensor
- 14: Strahlengang
- 14a: Anfangsabschnitt
- 14b: Endabschnitt
- 15: Spiegel
- 16: Linse
- 17: Lichtquelle
- 18: Linse
- 19: Blende
- 20: Fenster

## Patentansprüche

1. Stechsystem mit
Testelementen (5),
Stechelementen (3), denen jeweils ein Testelement (5) zugeordnet ist, und einem Handgerät, das einen Halter (7), der bei einem Stich ein Stechelement (3) und ein ihm zugeordnetes Testelement (5) hält, einen Stechantrieb (8), der bei einem Stich den Halter (7) aus einer Ausgangsposition in eine Einstichposition und zurück in die Ausgangsposition bewegt, einen lichtempfindlichen Sensor (13) zur Untersuchung einer von einem Testelement (5) aufgenommenen Körperflüssigkeitsprobe und eine Lichtquelle (17) zum Beleuchten des Testelements (5) aufweist,
einem Gehäuse (11), in dem der Sensor (13) angeordnet ist,
**dadurch gekennzeichnet, dass** der Halter (7) in seiner Ausgangsposition an der Außenseite des Gehäuses (11) anliegt, in dem der Sensor angeordnet ist.

2. Stechsystem nach Anspruch 1, **dadurch gekennzeichnet, dass** das Gehäuse (11) an seiner Außenseite mindestens eine Führung (12a, 12b) aufweist, die bei einer Stichbewegung den Halter (7) führt.

3. Stechsystem nach einem der vorstehenden Ansprüche, **dadurch gekennzeichnet, dass** in dem Gehäuse (11) wenigstens eine Linse (16, 18) angeordnet ist.

4. Stechsystem nach einem der vorstehenden Ansprüche, **dadurch gekennzeichnet, dass** in dem Gehäuse(11) wenigstens ein Spiegel (15) angeordnet ist.

5. Stechsystem nach einem der vorstehenden Ansprüche, **dadurch gekennzeichnet, dass** das Gehäuse (11) ein Fenster (11a) aufweist, an dem ein von dem Halter (7) gehaltenes Testelement (5) anliegt, wenn der Halter (7) in seiner Ausgangsposition ist.

6. Stechelement nach einem der vorstehenden Ansprüche, **dadurch gekennzeichnet, dass** in dem Gehäuse (11) ein gewinkelter Strahlengang (14) verläuft.

7. Stechsystem nach einem der vorstehenden Ansprüche, **dadurch gekennzeichnet, dass** das Testelement (5) ein Testfeld mit Nachweisreagenzien zur photometrischen Konzentrationsbestimmung ist.

8. Stechsystem nach einem der vorstehenden Ansprüche, **dadurch gekennzeichnet, dass** die Stechelemente (3) einen Grundkörper aufweisen, der das ihnen zugeordnete Testelement (5) trägt.

9. Stechsystem nach einem der vorstehenden Ansprüche, **dadurch gekennzeichnet, dass** in dem Gehäuse (11) ein Anfangsabschnitt (14a) eines von einem zu untersuchenden Testelement (5) ausgehenden Strahlengangs (14) quer zur Stichrichtung, in welcher der Halter (7) bei einem Stich bewegt wird, verläuft.

10. Stechsystem nach einem der vorstehenden Ansprüche, **dadurch gekennzeichnet, dass** in dem Gehäuse (11) ein Endabschnitt (14b) eines von einem zu untersuchenden Testelements (5) ausgehenden Strahlengangs (14) entlang der Stichrichtung, in welcher der Halter (7) bei einem Stich bewegt wird, verläuft.

11. Stechsystem nach einem der vorstehenden Ansprüche, **dadurch gekennzeichnet, dass** das Gehäuse (11) einen von dem Testelement (5) zu dem Sensor (13) führenden Strahlengang (14) von Störlicht abschirmt.

12. Stechsystem nach einem der vorstehenden Ansprüche, **dadurch gekennzeichnet, dass** die Lichtquelle (17) in dem Gehäuse (11) angeordnet ist.

13. Stechsystem nach einem der vorstehenden Ansprüche, **gekennzeichnet durch** ein Trägerband (2), das die Stechelemente (3) trägt.

14. Stechsystem nach einem der vorstehenden Ansprüche, **dadurch gekennzeichnet, dass** der Halter (7) zwei relativ zueinander bewegliche Halteelemente (7a, 7b) aufweist, zwischen denen ein Stechelement (3) gehalten wird.

15. Stechsystem nach einem der vorstehenden Ansprüche, **dadurch gekennzeichnet, dass** sich das Gehäuse (11) in Stichrichtung verjüngt.

## Claims

1. A lancing system, comprising
test elements (5),
lancing elements (3), test elements (5) each assigned to a lancing element, and
a hand-held device, comprising a holder (7), which during a puncture holds a lancing element (3) and a test element (5) assigned to it, a lancing drive (8), which during a puncture moves the holder (7) out of a starting position into a piercing position and back into the starting position, a light-sensitive sensor (13) for analyzing a body fluid sample taken up by a test element (5), and a light source (17) for illuminating the test element (5),
a housing (11) in which the sensor (13) is arranged,
**characterized in that** the holder (7) in its starting position touches the housing (11) in which the sensor (13) is arranged.

2. The lancing system according to claim 1, **characterized in that** the housing (11) comprises on its outside at least one guide (12a, 12b), which guides the holder (7) during a puncture movement.

3. A lancing system according to any one of the preceding claims, **characterized in that** at least one lens (16, 18) is disposed in the housing (11).

4. A lancing system according to any one of the preceding claims, **characterized in that** at least one mirror (15) is disposed in the housing (11).

5. A lancing system according to any one of the preceding claims, **characterized in that** the housing (11) comprises a window (11 a), which a test element (5) held by the holder (7) touches when the holder (7) is in the starting position.

6. A lancing system according to any one of the preceding claims, **characterized in that** an angled beam path (14) runs in the housing (11).

7. A lancing system according to any one of the preceding claims, **characterized in that** the test element (5) is a test field containing detection reagents for photometric concentration determination.

8. A lancing system according to any one of the preceding claims, **characterized in that** the lancing elements (3) have a base body, which carries the test element (5) assigned to them.

9. A lancing system according to any one of the preceding claims, **characterized in that** a starting section (14a) of a beam path (14) originating from the test element (5) to be analyzed runs in the housing (11) transversely to the puncture direction in which the holder (7) is moved during a puncture.

10. A lancing system according to any one of the preceding claims, **characterized in that** an end section (14b) of a beam path (14) originating from the test element (5) to be analyzed runs in the housing (11) along the puncture direction in which the holder (7) is moved during a puncture.

11. A lancing system according to any one of the preceding claims, **characterized in that** the housing (11) shields a beam path (14) which leads from the test element (5) to the sensor (13), from interfering light.

12. A lancing system according to any one of the preceding claims, **characterized in that** the light source (17) is disposed in the housing (11).

13. A lancing system according to any one of the preceding claims, **characterized by** a carrier tape (2) which carries the lancing elements (3).

14. A lancing system according to any one of the preceding claims, **characterized in that** the holder (7) comprises two holding elements (7a, 7b) that can be moved relative to one another, between which a lancing element (3) is held.

15. A lancing system according to any one of the preceding claims, **characterized in that** the housing (11) is tapered in the puncture direction.

## Revendications

1. Système piqueur comprenant
des éléments de test (5),
des éléments piqueurs (3) auxquels est associé respectivement un élément de test (5), et
un appareil à main, lequel comporte un support (7) qui, lors d'une piqûre, maintient un élément piqueur (3) et un élément de test (5) qui lui est associé, un entraînement (8) qui, lors d'une piqûre, déplace le support (7) d'une position de départ vers une position piqueur et le ramène à la position de départ, un capteur (13) sensible à la lumière pour l'analyse d'un échantillon de liquide corporel absorbé par un élément de test (5), et une source de lumière (17) pour éclairer l'élément de test (5),
un boîtier (11) dans lequel est placé le capteur (13),
**caractérisé en ce que** le support (7) est appliqué dans sa position de départ contre la face extérieure du boîtier (11) dans lequel est placé le capteur (13).

2. Système piqueur selon la revendication 1, **caractérisé en ce que** le boîtier (11) présente sur sa face extérieure au moins un guide (12a, 12b) qui dirige le support (7) lors d'un mouvement piqueur.

3. Système piqueur selon l'une des revendications précédentes, **caractérisé en ce qu**'au moins une lentille (16, 18) est placée dans le boîtier (11).

4. Système piqueur selon l'une des revendications précédentes, **caractérisé en ce qu'au** moins un miroir (15) est placé dans le boîtier (11).

5. Système piqueur selon l'une des revendications précédentes, **caractérisé en ce que** le boîtier (11) comporte une fenêtre (11 a) contre laquelle un élément de test (5) maintenu par le support (7) est appliqué lorsque le support (7) est dans sa position de départ.

6. Système piqueur selon l'une des revendications précédentes, **caractérisé en ce qu'**un trajet de rayon (14) faisant un angle passe dans le boîtier (11).

7. Système piqueur selon l'une des revendications précédentes, **caractérisé en ce que** l'élément de test (5) est une zone de test avec des réactifs de détection pour la détermination photométrique de concentration.

8. Système piqueur selon l'une des revendications précédentes, **caractérisé en ce que** les éléments piqueurs (3) ont un corps de base qui porte l'élément de test (5) qui leur est associé.

9. Système piqueur selon l'une des revendications précédentes, **caractérisé en ce que** dans le boîtier (11), une partie de début (14a) d'un trajet de rayon (14) partant d'un élément de test (5) à analyser s'étend transversalement au sens de la piqûre, dans lequel le support (7) est déplacé lors d'une piqûre.

10. Système piqueur selon l'une des revendications précédentes, **caractérisé en ce que** dans le boîtier (11), une partie finale (14b) d'un trajet de rayon (14) partant d'un élément de test (5) à analyser s'étend selon le sens de la piqûre, dans lequel le support (7) est déplacé lors d'une piqûre.

11. Système piqueur selon l'une des revendications précédentes, **caractérisé en ce que** le boîtier (11) protège de la lumière parasite un trajet de rayon (14) allant de l'élément de test (5) vers le capteur (13).

12. Système piqueur selon l'une des revendications précédentes, **caractérisé en ce que** la source de lumière (17) est placée dans le boîtier (11).

13. Système piqueur selon l'une des revendications précédentes, **caractérisé par** une bande support (2) portant les éléments piqueurs (3).

14. Système piqueur selon l'une des revendications précédentes, **caractérisé en ce que** le support (7) présente deux éléments de support (7a, 7b) déplaçables l'un vers l'autre, entre lesquels est maintenu un élément piqueur (3).

15. Système piqueur selon l'une des revendications précédentes, **caractérisé en ce que** le boîtier (11) s'amincit dans le sens de la piqûre.
